# EUROPEAN PATENT SPECIFICATION

(11) **EP 0 502 997 B1**
(45) Date of publication and mention of the grant of the patent: **03.05.1995**
(21) Application number: 91901503.2
(22) Date of filing: 29.11.1990
(51) Int. Cl.: A61K 38/21

(54) **Use of gamma-interferon for the manufacture of a medicament in the treatment of steroid dependent asthma**
Verwendung von Gamma-Interferon zur Herstellung eines Medikaments für die Behandlung von Steroid-abhängigem Asthma
Utilisation d'interféron-gamma dans la fabrication d'un médicament pour le traitement de l'asthme stéroide-dépendant

(30) Priority: 01.12.1989 US 444763
(43) Date of publication of application: 16.09.1992
(73) Proprietor: CHILDREN'S MEDICAL CENTER CORPORATION, Boston Massachusetts 02115 (US)
(72) Inventor: LEUNG, Donald, Y., M., Chestnut Hill, MA 02167 (US); Geha, Raif, S., Belmont, MA 02178 (US)
(74) Representative: Holdcroft, James Gerald, Dr.
(86) International application number: US9006971
(87) International publication number: WO9107984

(56) References cited:
- EP-A- 0 181 455
- WO-A-87/01288
- WO-A-87/07842

## Description

Steroid-dependent asthma is a severe form of IgE-mediated extrinsic (i.e., allergic) asthma. This type of asthma requires the frequent or constant use of systemic steroids to control the asthma symptoms.

While it is known to treat certain forms of asthma (e.g. salbutamol-responsive asthma) with gamma interferon (gIFN) (see e.g. EP A 181 455, WO A 8701288 and WO A 8707842), steroid-dependent asthma is a distinct disease which has not previously been known to respond to gIFN.

### Summary of the Invention

The present invention relates to the use of gamma interferon for the manufacture of a medicament for treating an individual having steroid-dependent asthma.

IFN-γ can be used as an adjunct in the treatment of allergic disorders. For example, IFN-γ treatment can be combined with treatment with one or more anti-allergic drugs, bronchodilators, cytokines or immunomodulators.

IFN-γ, used alone or as an adjunct, is a particularly effective drug for treating chronic, severe steroid-dependent asthma. The dosage level of gamma interferon is from 0.01 mg/m² to 0.1 mg/m² per day, for example 0,05 mg/m² (m² refers to square meters of total skin surface of the individual).

### Detailed Description of the Invention

In the present invention, patients afflicted with steroid-dependent asthma, are treated with IFN-γ.

The present use involves treating individuals by administering to the individual an amount of IFN-γ sufficient to reduce, ameliorate or eliminate the clinical symptoms of the disease.

In the present use, IFN-γ can be administered orally, by subcutaneous or other injection, intravenously, parenterally, transdermally or via an implanted reservoir or a sustained-release drug delivery device containing IFN-γ. The form in which the drug will be administered (e.g., powder, capsule, solution, emulsion) will depend upon the route by which it is administered.

The quantity of IFN-γ to be administered will depend in part on consideration of the individual's size, the severity of the symptoms to be treated and the result sought.

In general, the IFN-γ or composition containing IFN-γ is administered to an individual periodically as necessary to improve symptoms of the disease being treated. The length of time during which the drug is administered and the dosage will depend upon, inter alia, the type and severity of the symptoms and the physical condition of the individual being treated.

IFN-γ from any source can be used in the present method, including IFN-γ isolated from naturally-occurring sources and recombinant IFN-γ (rIFN-γ). As used herein, IFN-γ includes all proteins, peptides and polypeptides which are characterized by the biological activity of IFN-γ, for example, natural and recombinant IFN-γ or derivatives thereof. These include IFN-γ-like compounds from a variety of sources such as natural IFN-γ, recombinant IFN-γ and synthetic IFN-γ or combinations thereof. For example, IFN-γ useful in the present use includes natural IFN-γ produced in vitro by established or transformed cell lines and natural IFN-γ produced in vitro by a variety of cells in response to interferon inducers. IFN-γ useful in the present method also includes IFN-γ produced by cloning and expression of various host/vector systems using recombinant DNA technology. Recombinant IFN-γ is particularly useful because it is readily available and cost-effective.

In another embodiment IFN-γ or rIFN-γ can be used in adjunctive treatment combined with other drugs for reducing, ameliorating or eliminating the symptoms. IFN-γ is administered in conjunction with other drugs, including anti-allergy drugs (e.g., antihistamines), topical or systemic steroids, bronchodilators (e.g., theophylline), beta-adrenergic drugs, immunomodulators (e.g., cyclosporin, methotrexate) or cytokines (e.g., TNF, TGF-β, IFN-α, IL-2).

The composition to be administered can optionally include, in addition to IFN-γ or rIFN-γ, other components. The components included in a particular composition are determined primarily by the manner in which the composition is to be administered. In the case of adjunctive treatment with IFN-γ, IFN-γ is administered either concurrently with or in close temporal proximity to one or more drugs. For example, a composition to be administered in liquid form, by injection or other method, can include the IFN-γ or rIFN-γ, an adjunctive drug, if appropriate, and optionally, a physiologically compatible solvent (e.g., PBS, isotonic saline, water, dextrose), emulsifying agents or coloring agents. A composition to be administered orally can include in addition to IFN-γ, a filler (e.g., lactose) a binder (e.g., carboxymethyl cellulose, gelatin) an adjuvant, flavoring agent, coloring agent or coating material.

The present invention is useful in the treatment of chronic, severe steroid-dependent asthma.

### EXEMPLIFICATION

### MATERIALS AND METHODS

### Study Design

Recombinant INF-γ (Genentech, South San Francisco, CA; specific activity approximately 2 x 10⁷ U per milligram of protein [referenced to the National Institutes of Health interferon standard Gg23901530]) was supplied as an endotoxin-free lyophil, and reconstituted in distilled water. The study protocol consisted of two parts.

Part I was designed primarily to determine the maximum dose of rIFN-γ that would be well tolerated in this patient population. In this part, 14 patients were treated with rIFN-γ at three successive dose levels (DL): DL1 was 0.01 mg/m², DL2 was 0.05 mg/m², DL3 was 0.1 mg/M². Each dose was administered once daily by subcutaneous injection for five days with two days off between each DL. Clinical condition, routine laboratory studies, [what are routine laboratory studies?] and IgE production were evaluated at the time of screening, on days 1 and 5 of each DL, then 3 days off treatment.

In part II, 8 patients received rIFN-γ at DL2 (0.05 mg/m²) daily for 6 weeks. Clinical condition, routine laboratory studies, and IgE production were evaluated at the time of screening and on days 7, 21, and 42. Nine patients, one from part I and 8 from part II, were placed on maintenance therapy with rIFN-γ for the purpose of gathering long term tolerance data and to examine effects of long term rIFN-γ administration on serum IgE levels.

### Patients and Assessment of their Disease Activity

Twenty-two patients with chronic, severe atopic dermatitis (AD) a common inflammatory skin disease, were entered into a phase 1/pilot study to determine the tolerance of rIFN-γ in this patient population. Eligibility for this study required that the patient be 5 years of age or older, and have had a diagnosis of active chronic AD of at least 3 months duration. Diagnostic criteria for AD were those used by Hanifin and Rajka. Hanifin and Rajka, Acta Dermatol. Venereol., 92(supp.):44-47 (1980). In addition, all patients had to have an elevated serum IgE level (greater than 2 standard deviations above the mean for age) and a personal or family history of allergic respiratory disease or AD. Patients had to have a clinical severity score of 6 or greater (including a score of 2 or more for both erythema and pruritus) within 1 week prior to entry. The total clinical severity score (0-15) was defined as the sum of the individual scores, graded as 0 (none), 1 (mild), 2 (moderate) and 3 (severe), for each of five parameters: pruritus, erythma, edema/population, excoriation and scaling/dryness.

The extent of skin disease was estimated using the rule of nines. Barkin and Burrington, In: Current Pediatric Diagnosis and Treatment, C.H. Kempe et al., eds., p. 207, Appleton and Lange, Norwalk (1987). All patients had to have involvement of at least 20% of their body surface area.

The clinical and laboratory features of the patients entered into this protocol are summarized in Table I.

**Table I**

| Characteristics of AD Patients Entered into Open Label Study of Gamma Interferon Treatment¹ | | |
|---|---|---|
| Characteristic | Part I | Part II |
| Sex (M/F) | 9/5 | 4/4 |
| Median Age (Yrs) | 33 | 25 |
| Serum IgE (IU/ml) | 10,500(680-59,100) | 6420(573-65,700) |
| Duration of AD(Yrs) | 28 (3-47) | 18 (13-51) |
| % Body Involvement | 72 (40-100) | 81 (27-100) |
| Total Clinical Severity² | 13 (9-15) | 14 (9-15) |

All patients had a history of chronic severe AD which had not responded well to previous treatment with standard therapy including the use of emollients, potent topical steroids, and anti-histamines. Most patients had required past hospitalizations or systemic steriods for control of their skin disease.

### Statistical Analysis

For part I, changes between day 1 and day 5 of each DL for serum IgE, spontaneous IgE synthesis and total clinical severity were analyzed using a two-sided sign test or Wilcoxon signed-rank test. Changes within each DL for each patients were also similarly compared to baseline changes between screening to DL1, day 1. In part II, the changes over time for each patient in serum IgE, spontaneous IgE synthesis and total clinical severity were statistically assessed using the two-sided sign test or Wilcoxon signed-rank test.

### RESULTS

Fourteen AD patients entered into part I of the protocol. All 14 patients completed DL1 without any adverse reactions. On DL2, three of the 14 patients had transient headaches or myalgias (4% of doses). One patient dropped out of the study at DL2 for personal reasons. On DL3, nine of the 13 remaining patients experienced transient headache, malaise, fever/chills, myalgia, nausea (25% of doses). The only significant lab abnormality noted was a dose dependent reversible decrease in neutrophil count at each DL. No patient, however, developed absolute neutropenia (<1000 neutrophils per cu mm) during the study.

One of the 22 patients treated in Part 1 of the study was afflicted with chronic severe, steroid-dependent asthma in addition to atopic dermatitis. This patient had required treatment with systemic steroids for at least 6 years prior to the study. This patient showed a significant improvement in asthma symptoms and was able to discontinue systemic steroid treatment after treatment with IFN-γ. This patient, in prophylactic or maintenance therapy with IFN-γ, has not required systemic steroids for over one year.

## Claims

1. Use of gamma interferon for the manufacture of a medicament for treating an individual having steroid dependent asthma.

2. Use according to Claim 1 wherein the gamma interferon is recombinant gamma interferon.

3. Use according to Claim 1 wherein the dosage level of gamma interferon is from 0.01 mg/m² to 0.1 mg/m² per day.

4. Use according to Claim 3 wherein the dosage level of gamma interferon is about 0.05 mg/m² per day.

5. Use according to Claim 1 in which at least one additional drug is used in conjunction with gamma interferon and is selected from a group consisting of: anti-allergy drugs, steroids, bronchodilators, beta-adrenergic agonists, immunomodulators and cytokines.

## Patentansprüche

1. Verwendung von Gamma-Interferon zur Herstellung eines Medikaments für die Behandlung eines Individuums, das an Steroid-abhängigem Asthma leidet.

2. Verwendung nach Anspruch 1, wobei das Gamma-Interferon rekombinantes Gamma-Interferon ist.

3. Verwendung nach Anspruch 1, wobei das Dosierungsniveau von Gamma-Interferon 0,01 mg/m² bis 0,1 mg/m² pro Tag ist.

4. Verwendung nach Anspruch 3, wobei das Dosierungsniveau von Gamma-Interferon etwa 0,05 mg/m² pro Tag ist.

5. Verwendung nach Anspruch 1, wobei mindestens ein zusätzliches Arzneimittel zusammen mit Gamma-Interferon angewendet wird und dieses aus der Gruppe
Antiallergika, Steroide, Bronchodilatoren, betaadrenergische Agonisten, Immunmodulatoren und Zytokine
ausgewählt ist.

## Revendications

1. Utilisation d'interféron gamma pour la fabrication d'un médicament pour traiter un individu atteint d'asthme stéroïde-dépendant.

2. Utilisation selon la revendication 1, dans laquelle l'interféron gamma est un interféron gamma recombinant.

3. Utilisation selon la revendication 1, dans laquelle la dose d'interféron gamma est de 0,01 mg/m² à 0,1 mg/m² par jour.

4. Utilisation selon la revendication 3, dans laquelle la dose d'interféron gamma est d'environ 0,05 mg/m² par jour.

5. Utilisation selon la revendication 1, dans laquelle on utilise au moins un médicament supplémentaire en conjonction avec l'interféron gamma, que l'on choisit dans le groupe constitué par les médicaments anti-allergiques, les stéroïdes, les bronchodilatateurs, les agonistes β-adrénergiques, les immunomodulateurs et les cytokines.
